# EUROPÄISCHE PATENTSCHRIFT

(11) **EP 0 893 444 B1**
(45) Veröffentlichungstag und Bekanntmachung des Hinweises auf die Patenterteilung: **02.11.2005**
(21) Anmeldenummer: 98112967.9
(22) Anmeldetag: 13.07.1998
(51) Int. Cl.: C07D 319/20, C07D 319/22, C07C 43/225, C07C 43/23, C07C 43/205, C07C 47/575

(54) **Verfahren zur Herstellung von 2,2,3,3-Tetrafluor-1,4-benzodioxanen, neue o-(2-Brom-1,1,2,2,-tetrafluorethoxy)-phenole und neue 2-Brom-1,1,2,2-tetrafluorethoxy-gruppen enthaltende Phenylether**
Process for reparing 2,2,3,3-tetrafluoro-1,4-benzodioxanes, new o-(2-bromo-1,1,2,2,-tetrafluoroethoxy)-phenols and new2-bromo-1,1,2,2-tetrafuoroethoxy-phenylethers
Procédé pour la préparation de 2,2,3,3-tétrafluoro-1,4-benzodioxannes, nouveaux o-(2-bromo-1,1,2,2,-tétrafluoroéthoxy)-phénoles et nouveaux 2-bromo-1,1,2,2-tétrafluoroéthoxy-phényléthers

(30) Priorität: 24.07.1997 DE 19731785
(43) Veröffentlichungstag der Anmeldung: 27.01.1999
(73) Patentinhaber: LANXESS Deutschland GmbH, 51369 Leverkusen (DE)
(72) Erfinder: Popkova, Vera Yakovlevna Prof. Dr., 117421 Moscow (RU); Marhold, Albrecht Dr., 51373 Leverkusen (DE)

(56) Entgegenhaltungen:
- EP-A- 0 052 558
- EP-A- 0 124 002
- DE-A- 2 819 788
- US-A- 4 737 509
- DELLA COR ET AL.: "Ring-closure reactions...." J.CHEM.SOC.,PERKIN TRANS. 2, Bd. 12, 1980, Seiten 1774-1777, XP002083515

## Beschreibung

Die vorliegende Erfindung betrifft ein Verfahren zur Herstellung von 2,2,3,3-Tetrafluor-1,4-benzodioxanen, neue Zwischenprodukte die in diesem Verfahren auftreten und Verfahren zu deren Herstellung. 2,2,3,3-Tetrafluor-1,4-benzodioxane werden als Zwischenprodukte bei der Herstellung von Wirkstoffen für Pharmazie und Landwirtschaft benötigt (siehe z.B. DE-A 37 16 652, DE-A 19 501 367, DEA 4 415 435 und DE-A 4 217 725).

Aus der Literatur ist ein dreistufiges Verfahren zur Herstellung von unsubstituiertem 2,2,3,3-Tetrafluor-1,4-benzodioxan bekannt, das von Brenzkatechin ausgeht. Im ersten Schritt wird jenes mit Trifluorchlorethen zu 2,2,3-Trifluor-1,4-benzodioxan umgesetzt. Dieses muß dann zu 2,2,3-Trifluor-3-chlor-1,4-benzodioxan chloriert werden. Im dritten Schritt wird schließlich ein Chlor/Fluor-Austausch durchgeführt. Dieses Verfahren hat eine Reihe von Nachteilen. Der letzte Schritt ist nur unbefriedigend durchführbar. Die Fluorierung durch Disproportionierung in Gegenwart von Antimon(V)-chlorid ist technisch aufwendig und erfordert die Rückführung von 50 Gew.-% des Materials (siehe DE-A 3 315 147). Die alternative Gasphasenreaktion mit Fluorwasserstoff bei 350°C (siehe EP-A 623 609) ist mit schlechter Reproduzierbarkeit durchführbar und wäre technisch nur unter großem Aufwand realisierbar. Wenig vorteilhaft sind die korrosiven Medien wie beim Einsatz von Chlorgas die Chlorwasserstofffreisetzung beim zweiten Schritt sowie gegebenenfalls von Fluorwasserstoff beim dritten. Darüber hinaus läßt sich dieses Verfahren nicht ohne weiteres auf substituierte Brenzkatechine übertragen. Beispielsweise werden Alkylsubstituenten am Benzolkern unter den angewendeten Chlorierungsbedingungen auch chloriert.

Es wurde nun ein Verfahren zur Herstellung von 2,2,3,3-Tetrafluor-1,4-benzodioxanen der Formel 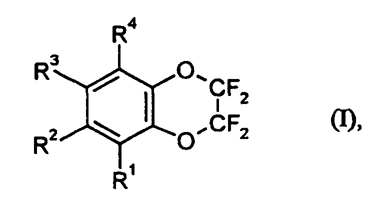 in welcher
- R¹ bis R⁴: unabhängig voneinander jeweils für Wasserstoff, Halogen oder C₁-C₆-Alkyl stehen,
wobei zwei benachbarte Reste von R¹ bis R⁴ auch gemeinsam für - (CH₂)₃-, -(CH₂)₄-, -(CH₂)₅- oder eine gegebenenfalls durch Halogen oder C₁-C₆-Alkyl substituierte Kette -CH=CH-CH=CH- stehen können,
und wobei ein oder zwei der Reste R¹ bis R⁴ darüber hinaus auch für Nitro, Cyano, -CO-R⁶ oder -C(OR⁷)₂R⁶ stehen können, mit R⁶ = Wasserstoff oder C₁- C₆-Alkyl und R⁷ = C₁-C₆-Alkyl oder beide Reste gemeinsam = -CH₂CH₂-, -CH₂CH(CH₃)-, CH₂CH(C₂H₅)- oder -CH(CH₃)CH(CH₃)-
gefunden, das dadurch gekennzeichnet ist, daß man o-(2-Brom-tetrafluorethoxy)-phenole der Formel 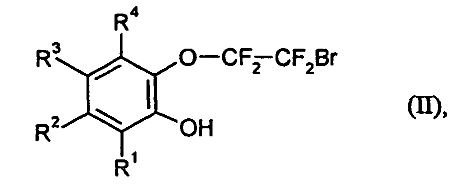 in welcher R¹ bis R⁴ die bei Formel (I) angegebene Bedeutung haben,
in Gegenwart eines Säurebindemittels cyclokondensiert.

Die Verbindungen der Formel (II) sind neu und auch Gegenstand der vorliegenden Erfindung.

Weiterhin wurde ein Verfahren zur Herstellung der Phenole der Formel (II) gefunden, das dadurch gekennzeichnet ist, daß man Verbindungen der Formel (III) 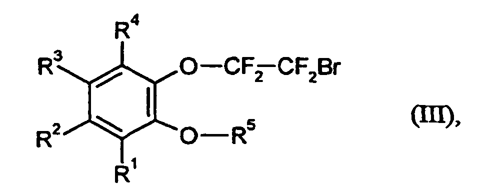 in welcher
- R¹ bis R⁴: die bei Formel (I) angegebene Bedeutung haben und
- R⁵: für C₁-C₆-Alkyl oder Benzyl steht,
einer Etherspaltung unterwirft.

Die Verbindungen der Formel (III) sind, mit Ausnahme von 1-(2-Brom-1,1,2,2-tetrafluorethoxy)-2-methoxy-4-nitro-benzol, dessen Herstellung in der Literatur aber nicht beschrieben ist (siehe US-A 4 737 509), neu und auch Gegenstand der vorliegenden Erfindung.

Weiterhin wurde ein Verfahren zur Herstellung der Verbindungen der Formel (III) gefunden, das dadurch gekennzeichnet ist, daß man Verbindungen der Formel (IV) 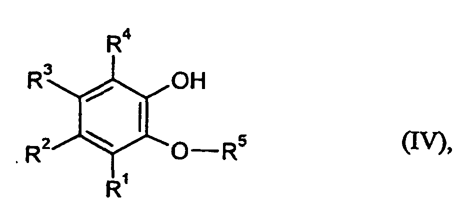 in welcher R¹ bis R⁴ die bei Formel (I) und R⁵ die bei Formel (III) angegebenen Bedeutungen haben,
mit 1,2-Dibrom-1,1,2,2-tetrafluorethan in Gegenwart eines Säurebindemittels umsetzt.

Die Verbindungen der Formel (IV) sind z.T. kommerziell erhältlich oder nach bekannten Methoden oder analog dazu herstellbar. 1,2-Dibrom-1,1,2,2-tetrafluorethan ist als Feuerlöschmittel kommerziell verfügbar.

Die erfindungsgemäß herstellbaren Verbindungen sind durch die Formeln (I), (II) und (III) definiert, die erfindungsgemäßen Verbindungen durch die Formeln (II) und (III), wobei jedoch bei Formel (II) die Verbindung 1-(2-Brom-1,1,2,2-tetrafluorethoxy)-2-methoxy-4-nitro-benzol ausgenommen ist.

Die in den Formeln (I), (II), (III) und (IV) verwendeten Symbole haben folgende bevorzugten Bedeutungen:
R¹ bis R⁴ Wasserstoff Fluor, Chlor, Brom oder C₁-C₄-Alkyl,
   wobei zwei benachbarte Reste von R¹ bis R⁴ auch gemeinsam für - (CH₂)₃-, -(CH₂)₄-oder eine gegebenenfalls durch Fluor, Chlor, Brom oder C₁-C₄-Alkyl substituierte Kette -CH=CH-CH=CH- stehen können
   und wobei einer oder zwei der Reste R¹ bis R⁴ darüber hinaus auch für Nitro, Cyano, -CO-R⁶ oder -C(OR⁷)₂R⁶ stehen können.
   - R⁵: C₁-C₄-Alkyl oder Benzyl.
   - R⁶: Wasserstoff oder C₁-C₄-Alkyl.
   - R⁷: Methyl, Ethyl oder beide Reste R⁷ gemeinsam für -CH₂CH₂- oder -CH₂CH(CH₃).

Die in den Formeln (I), (II), (III) und (IV) verwendeten Symbole haben folgende besonders bevorzugten Bedeutungen:
- R¹ bis R⁴: Wasserstoff, Fluor, Chlor, Brom, Methyl, Ethyl, n-Propyl, Isopropyl, n-Butyl, Isobutyl, sec.-Butyl oder tert.-Butyl,
wobei zwei benachbarte Reste von R¹ bis R⁴ auch gemeinsam für -(CH₂)₃- oder eine gegebenenfalls durch Fluor, Chlor, Brom, Methyl, Ethyl, n-Propyl, Isopropyl, n-Butyl, Isobutyl, sec.-Butyl oder tert.-Butyl substituierte Kette - CH=CH- CH=CH- stehen können
und wobei einer der Reste R¹ bis R⁴ auch für Nitro, Cyano, -CO-R⁶ oder -C(OR⁷)₂R⁶ stehen kann.
- R⁵: Methyl, Ethyl, n-Propyl, Isopropyl, n-Butyl, Isobutyl, sec.-Butyl, tert.-Butyl oder Benzyl.
- R⁶: Wasserstoff Methyl, Ethyl, n-Propyl, Isopropyl, n-Butyl, Isobutyl oder tert.-Butyl.
- R⁷: Methyl oder Ethyl oder beide Reste R⁷ gemeinsam für -CH₂CH₂-.

Die in den Formeln (I), (II), (III) und (IV) verwendeten Symbole haben folgende ganz besondes bevorzugten Bedeutungen:
- R¹: Wasserstoff.
- R²: Wasserstoff, Methyl oder Ethyl.
- R³: Wasserstoff, Methyl, Ethyl, Chlor oder Formyl.
- R⁴: Wasserstoff, Methyl, Ethyl oder Formyl.
- R² und R³: -CH=CH-CH=CH-.
- R⁵: Methyl oder Ethyl.

Die oben aufgeführten allgemeinen oder in Vorzugsbereichen aufgeführten Definitionen können untereinander, also auch zwischen den jeweiligen Bereichen und Vorzugsbereichen beliebig kombiniert werden.

Bevorzugte, besonders bevorzugte und ganz besonders bevorzugte Verbindungen der Formeln (I), (II), (III) und (IV) sind solche, in denen eine Kombination der vorstehend so bezeichneten Bedeutungen vorliegt.

Kohlenwasserstoffreste wie Alkyl können jeweils geradkettig oder verzweigt sein.

Gegebenenfalls substituierte Reste können einfach oder mehrfach, z.B. bis zu dreifach, substituiert sein, wobei bei Mehrfachsubstitutionen die Substituenten gleich oder verschieden sein können.

Als Säurebindungsmittel für die Umsetzung von Verbindungen der Formel (IV) mit 1,2-Dibrom-1,1,2,2-tetrafluorethan kommen z.B. übliche anorganische und organische Basen in Frage. Hierzu gehören Alkalimetall- und Erdalkalimetallhydride, -hydroxide, -amide, -alkoholate, -acetate, -carbonate und -hydrogencarbonate, beispielsweise Natriumhydrid, Natrium-, Kalium- und Ammoniumhydroxid, Natriumamid, Lithiumdiisopropylamid, Natrium-methylat, Natrium-ethylat, Kalium-tert.-butylat, Natrium-, Kalium-, Calcium- und Ammoniumacetat, Natrium-, Kalium- und Ammoniumcarbonat, Natrium- und Kaliumhydrogencarbonat, und tertiäre Amine wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) und Diazabicycloundecen (DBU). Bevorzugt ist Kaliumcarbonat.

Diese Reaktion wird vorzugsweise in Gegenwart eines Verdünnungsmittels durchgeführt. Hierfür kommen z.B. dipolar-aprotische Lösungsmittel und deren Mischungen in Betracht. Beispielhaft seien genannt: Nitrite wie Acetonitril, Propionitril, n- und i-Butyronitril und Benzonitril, Amide wie Formamid, N,N-Dimethylformamid, N,N-Dimethylacetamid, N-Methylformanilid, N-Methylpyrrolidon und Hexamethylphosphorsäuretriamid, N-Oxide wie N-Methylmorpholin-N-oxid, Ester wie Methyl-, Ethyl- oder Butylacetat, Sulfoxide wie Dimethylsulfoxid und Sulfone wie Sulfolan. Bevorzugt ist Dimethylsulfoxid.

Die Temperatur kann bei dieser Reaktion in einem weiten Bereich variiert werden. Im allgemeinen arbeitet man bei 20 bis 130°C, bevorzugt bei 70 bis 120°C.

Pro Mol der Verbindung der Formel (IV) kann man z.B. 1 bis 3 Mol, bevorzugt 1,5 bis 2,5 Mol 1,2-Dibrom-1,1,2,2-tetrafluorethan, z.B. 1 bis 6 Äquivalente, bevorzugt 1 bis 4 Äquivalente Säurebindemittel und z.B. 600 bis 3000 ml, vorzugsweise 1500 bis 2500 ml Verdünnungsmittel einsetzen.

Diese Reaktion kann unter Normaldruck oder erhöhtem Druck durchgeführt werden. Vorteilhaft ist es in einem geschlossenen Gefäß zu arbeiten, wobei sich dann während der Reaktion ein Druck von beispielsweise bis zu 10 bar einstellen kann.

Bei dieser Reaktion entsteht in Abhängigkeit von den Substituenten und den Reaktionsbedingungen in geringen Mengen der entsprechende reduzierte 1,1,2,2-Tetrafluorethylether (V)

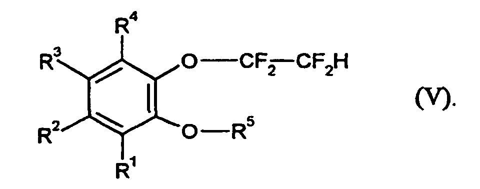

Die Reaktionsdurchführung, Aufarbeitung und Isolierung der Reaktionsprodukte kann nach allgemein üblichen Verfahren erfolgen. Die Verbindungen der Formeln (III) und (V) können gewünschtenfalls beispielsweise durch Destillation oder Säulenchromatographie getrennt werden. Vorzugsweise werden die Verbindungen der Formeln (III) und (V) jedoch nicht getrennt, sondern deren Gemisch weiter umgesetzt.

Die Etherspaltung von Verbindungen der Formel (III) zu Phenolen der Formel (II) kann man nach an sich bekannten Methoden durchführen. Man setzt beispielsweise wäßrige Halogenwasserstoffsäuren wie Iodwasserstoff, Bromwasserstoff oder Chlorwasserstoff ein und arbeitet gegebenenfalls in Gegenwart einer C₁-C₄-Carbonsäure wie Ameisen-oder Essigsäure als Löslichkeitsvermittler. Die Konzentration der Halogenwasserstoffsäure liegt vorzugsweise zwischen 20 Gew.-% und der Sättigungskonzentration. Man kann auch gasförmige Halogenwasserstoffsäure einsetzen und arbeitet dann vorzugsweise in einem Autoklaven. Im Falle des Einsatzes eines Benzylethers (Formel (III), R⁵ = Benzyl) wird die Etherspaltung bevorzugt durch katalytische Hydrierung durchgeführt. Als Katalysatoren kommen beispielsweise Raney-Nickel oder Palladium-auf-Kohle und als Lösungsmittel beispielsweise Methanol, Ethanol oder Ethylacetat in Frage.

Enthält die Verbindung der Formel (III) Acetal- bzw. Ketalsubstituenten (einer oder zwei der Reste R¹ bis R⁴ = -C(OR⁷)₂R⁶) so werden diese bei der Etherspaltung mit Säure ebenfalls gespalten und in den entspechenden Carbonylrest der Formel -CO-R⁶ gewandelt. Wenn das Zielprodukt der Synthesefolge ein Benzodioxan der Formel (I) ist, bei denen mindestens einer der Reste R¹ bis R⁴ für -C(OR⁷)₂R⁶ steht, ist es daher vorteilhaft, von einem entsprechenden einfach benzylgeschütztem Brenzkatechin der Formel (IV), in der R⁵ für Benzyl steht, auszugehen, und die Etherspaltung als katalytische Hydrierung durchzuführen. Natürlich kann man auch die Ketal- bzw. Acetalfunktion am Ende der Synthese aus der Carbonylfunktion -CO-R⁶ wieder oder erstmalig herstellen.

Die Temperatur kann bei der Etherspaltung in einem weiten Bereich variiert werden. Beispielsweise kann man bei der sauren Spaltung bei 30 bis 150°C, bevorzugt 50 bis 140°C arbeiten. Bei der katalytischen Hydrierung kann man beispielsweise bei 0 bis 50°C, bevorzugt bei Raumtemperatur, arbeiten.

Bei der sauren Etherspaltung ist die zu verwendende Säuremenge nach oben hin nicht kristisch. Pro Mol der Verbindung der Formel (III) setzt man zweckmäßigerweise mindestens die theoretische benötigte Menge von 1 Mol Halogenwasserstoffsäure ein. Bevorzugt werden Reaktanden und Hilfsmittel in solchen Mengen eingesetzt, die während der sauren Etherspaltung eine weitgehend homogene Lösung ergeben. Besonders vortelhaft setzt man wäßrige Bromwasserstoffsäure einer Konzentration von 30 bis 70 Gew.-% und Essigsäure einer Konzentration von 80 bis 100 Gew.-% im Gewichtsverhältnis 1:1,5 bis 1:3 ein, löst dabei zunächst die Verbindung der Formel (III) in der Essigsäure und fügt dann die Bromwasserstoffsäure hinzu.

Setzt man eine Verbindung der Formel (III) ein, die eine Verbindung der Formel (V) enthält, so enthält man ein o-Tetrafluorethoxyphenol der Formel (VI)

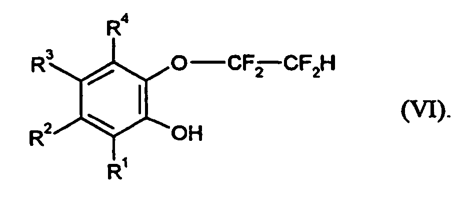

Die Reaktionsdurchführung, Aufarbeitung und Isolierung der Reaktionsprodukte kann nach üblichen Verfahren erfolgen. Verbindungen der Formeln (II) und (VI) können gewünschtenfallss beispielsweise durch Destillation oder Säulenchromatographie getrennt werden. Vorzugsweise werden die Verbindungen der Formeln (II) und (VI) jedoch nicht getrennt, sondern deren Gemisch weiter umgesetzt.

Die Cyclokondensation der Phenole der Formel (II) zu den Benzodioxanen der Formel (I) wird in Gegenwart eines Säurebindemittels durchgeführt. Als Säurebindemittel kommen z.B. diejenigen in Frage, die weiter oben für die Umsetzung von Verbindungen der Formel (IV) zu Verbindungen der Formel (III) angegeben wurden. Für die Cyclokondensation der Phenole der Formel (II) zu den Benzodioxanen der Formel (I) sind als Säurebindemittel Natriumhydroxid, Kaliumhydroxid, Natriummethylat und Kaliummethylat bevorzugt.

Diese Cyclokondensation kann gegebenenfalls in Gegenwart eines Verdünnungsmittels durchgeführt werden. Geeignete Verdünnungsmittel sind z.B. höhersiedende dipolar-aprotische Lösungsmittel wie Sulfone, insbesondere Sulfolan. Vorzugsweise wird ein Verdünnungsmittel eingesetzt.

Diese Cyclokondensation kann man z.B. bei 80 bis 180°C, vorzugsweise bei 100 bis 140°C durchführen.

Zur Durchführung dieser Cyclokondensation kann man z.B. pro Mol des Phenols der Formel (II) 1 bis 4 Äquivalente, vorzugsweise 1 bis 2 Äquivalente Säurebindemittel einsetzen.

Die Reaktionsdurchführung, Aufarbeitung und Isolierung der Reaktionsprodukte kann nach allgemein üblichen Verfahren erfolgen.

In einer bevorzugten Ansfuhrungsform geht man zweistufig vor. Zunächst stellt man durch Umsetzung des Phenols der Formel (II) mit einem Säurebindemittel aus der Reihe der Erdalkalihydroxide und der Alkalihydroxide, -alkoholate und -acetate bei wenig erhöhter Temperatur das entsprechende Phenolat her, indem man unter gegebenenfalls vermindertem Druck und gegebenenfalls durch Zusatz eines Verdünnungsmittels oder Azeotropbildners wie Toluol den Leichtsieder, z.B. Wasser, Alkohol oder Essigsäure, abdestilliert. Darauf gibt man, wenn noch nicht geschehen, das Verdünnungsmittel hinzu und führt durch Temperaturerhöhung die Cyclokondensation durch, wobei das gebildete 2,2,3,3-Tetrafluor-1,4-benzodioxan der Formel (I) meistens direkt unter vermindertem Druck abdestilliert werden kann. Gegebenenfalls in die Reaktion mit eingebrachtes o-Tetrafluorethoxyphenol der Formel (VI) verbleibt dabei als Phenolat im Sumpf.

Alle Verfahrensschritte werden, soweit nicht anders angegeben, üblicherweise unter Normaldruck durchgeführt. Es ist jedoch auch möglich, unter erhöhtem oder vermindertem Druck zu arbeiten.

Alle Verfahrensschritte können kontinuierlich oder diskontinuierlich durchgeführt werden, insbesondere die Cyclisierung.

Das erfindungsgemäße Verfahren erlaubt über den Stand der Technik hinaus auch die Herstellung substituierter 2,2,3,3-Tetralluor-1,4-benzodioxane auf direktem Wege in guten Ausbeuten. Es ist auch in technisch einfacher Weise durchführbar und vermeidet korrosive Medien wie Chlor und Fluorwasserstoff.

### Beispiele

Die mit GC bestimmten prozentualen Zusammensetzungen von Produkten und Edukten beziehen sich auf die Flächenverhältnisse (Fl. %) der Signale eines Flammenionisationsdetektors und sind deshalb im allgemeinen nicht identisch mit Mol- oder Gewichtsprozenten.

### Beispiel I-1

### Herstellung von 2,2,3,3-Tetrafluor-1,4-benzodioxan

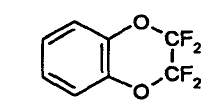

Eine Lösung von 10,16 g 2-(2-Brom-1,1,2,2-tetrafluorethoxy)-phenol in 20 ml trockenem Diethylether wurde tropfenweise zu einer gerührten Suspension von 2,68 g Kaliummethylat in 20 ml trockenem Diethylether gegeben. Nach dem Ende der exothermen Reaktion wurden Diethylether und Methanol bis zur Trockene abgedampft.

9,52 g des so erhaltenen trockenen Kaliumsalzes wurden im Vakuum (14 mbar) unter Rühren in 25 ml trockenem Sulfolan auf 125 bis 145°C erhitzt, wobei das übergehende Destillat in einer auf -78°C gekühlten Vorlage aufgefangen wurde. Es wurden 5,01g (82,8 % d.Th.) 2,2,3,3-Tetrafluor-1,4-benzodioxan erhalten. Das Produkt war gaschromatographisch einheitlich und die ¹H-, ¹⁹F-NMR- und MS-Spektren entsprachen denjenigen von gemäß der DE-A 3 314 147 hergestelltem 2,2,3,3-Tetrafluor-1,4-benzodioxan..

Das Erhitzen des trockenen Kaliumsalzes wurde auch kontinuierlich (Zugabe: 27 g im Verlauf einer Stunde) durchgeführt, wobei die gleichen Bedingungen eingehalten und pro Stunde 14,3 g 2,2,3,3-Tetrafluor-1,4-benzodioxan erhalten wurde.

### Beispiel 1-2

### Herstellung von 2,2,3,3-Tetrafluor-1,4-benzodioxan

Eine Lösung von 2,87 g Kaliumhydroxid in 10 ml Methanol wurde tropfenweise zu einer Lösung von 9,86 g 2-(2-Brom-1,1,2,2-tetrafluorethoxy)-phenol in 10 ml Methanol gegeben. Dann wurden Methanol und Wasser im Vakuum bis zur Trockene abgedampft. Das restliche Wasser wurde aus dem festen Rückstand durch Zugabe von Toluol und erneutem Eindampfen in Vakuum azeotrop entfernt.

7,89 g des so erhaltenen trockenen Kaliumsalzes wurden im Vakuum (15 bis 20 mbar) unter Rühren in 25 ml trockenem Sulfolan auf 140 bis 145°C erhitzt, wobei das übergehende Destillat in einer auf -78°C gekühlter Vorlage aufgefangen wurde. Es wurden 3,08 g (61,4 % d.Th.) 2,2,3,3-Tetrafluor-1,4-benzodioxan erhalten. Das Produkt war gaschromatographisch einheitlich und die ¹H-, ¹⁹F-NMR- und MS-Spektren entsprachen denjenigen von gemäß der DE-A 3 315 147 hergestelltem 2,2,3,3-Tetrafluor-1,4-benzodioxan.

### Beispiel 1-3

### Herstellung von 6-Formyl-2,2,3,3-tetrafluor-1,4-benzodioxan

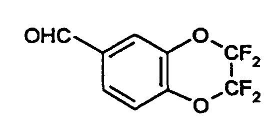

Analog Beispiel I-2 wurde eine Mischung der trockenen Kaliumsalze von 3-(2-Brom 1,1,2,2-tetrafluorethoxy)-4-hydroxybenzaldehyd (85 Fl.-%) und 3-(1,1,2,2-Tetrafluorethoxy)-4-hydroxybenzaldehyd (15 Fl.-%) hergestellt und 2,80 g hiervon bei 145°C/10 mbar in 20 ml trockenem Sulfolan erhitzt. Es wurden 1,62 g einer Mischung aus 13,4 Fl.-% 6-Formyl-2,2,3,3-tetrafluor-1,4-benzodioxan (12,7% d.Th.) und 86,6 Fl.-% Sulfolan erhalten (GC-Analyse). Das bei 100°C/20 mbar siedende gewünschte Produkt wurde durch Destillation rein erhalten. Es entsprach gemäß der DE-A 3 716 652 erhaltenen 6-Formyl-2,2,3,3-tetrafluor-1,4-benzodioxan.

### Beispiel I-4

### Herstellung von 6-Chlor-2,2,3,3-tetrafluor-1,4-benzodioxan

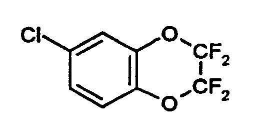

10,0 g einer Mischung von 77 Fl.-% 1-Hydroxy-2-(2-brom-1,1,2,2-tetrafluorethoxy)-4-chlorbenzol und 23 Fl.-% 1-Hydroxy-2-(1,1,2,2-tetrafluorethoxy)-4-chlorbenzol wurden mit 3,48 g Kaliumhydroxid in 30 ml Sulfolan für 9 Stunden bei 120°C gerührt. Danach wurde die Reaktionsmischung auf 20°C abgekühlt und die bei 0,3 mbar und 20 bis 60°C flüchtigen Produkte in einer auf -78°C gekühlte Vorlage aufgefangen. Es wurden 2,56 g einer Mischung von 94,5 Fl.-% 6-Chlor-2,2,3,3-tetrafluor-1,4-benzodioxan (41,9 % d.Th.) und 5,5 Fl.-% 6-Chlor-2,3,3-trifluor-1,4-benzodioxan erhalten (GC-, ¹H-, ¹⁹F-NMR- und GC-MS-Analyse). Das bei 75°C/238 mbar siedende gewünschte Produkt wurde durch Destillation rein erhalten.

| C/H-Analyse[%] | C | H |
|---|---|---|
| ber. für C₈H₃O₂ClF₄ | 39,61 | 1,247 |
| gef. | 39,6 | 1,3 |

### Beispiel I-5

### Herstellung von 2,2,3,3-Tetrafluor-6-methyl-1,4-benzodioxan

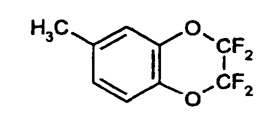

7,0 g einer Mischung von 83 Fl-% 4-(2-Brom-1,1,2,2-tetrafluorethoxy)-1-methyl-3-hydroxybenzol und 17 Fl.-% 4-(1,1,2,2-Tetrafluorethoxy)-1-methyl-3-hydroxybenzol wurden mit 1,9 g Natriumhydroxid in 30 ml Sulfolan für 30 Stunden bei 120°C gerührt. Danach wurde die Reaktionsmischung auf 20°C abgekühlt und die bei 0,3 mbar und 20 bis 60°C flüchtigen Produkte in einer auf -78°C gekühlte Vorlage aufgefangen. Es wurden 2,5 g einer Mischung von 90 Fl.-% 2,2,3,3-Tetrafluor-6-methyl-1,4-benzodioxan (52,8 % d.Th.) und 10 Fl.-% 2,2,3-Trifluor-6-methyl-1,4-benzodioxan erhalten (GC-, ¹H-, ¹⁹F-NMR- und GC-MS-Analyse). Das bei 98°C/238 mbar siedende gewünschte Produkt wurde durch Destillation rein erhalten.

| C/H-Analyse [%] | C | H |
|---|---|---|
| ber. für C₉H₆O₂F₄ | 48,66 | 2,72 |
| gef | 49,2 | 2,9 |

Die oben beschriebenen Beispiele und weitere, die analog zu diesen durchgeführt wurden, sind in Tabelle I zusammengefaßt.

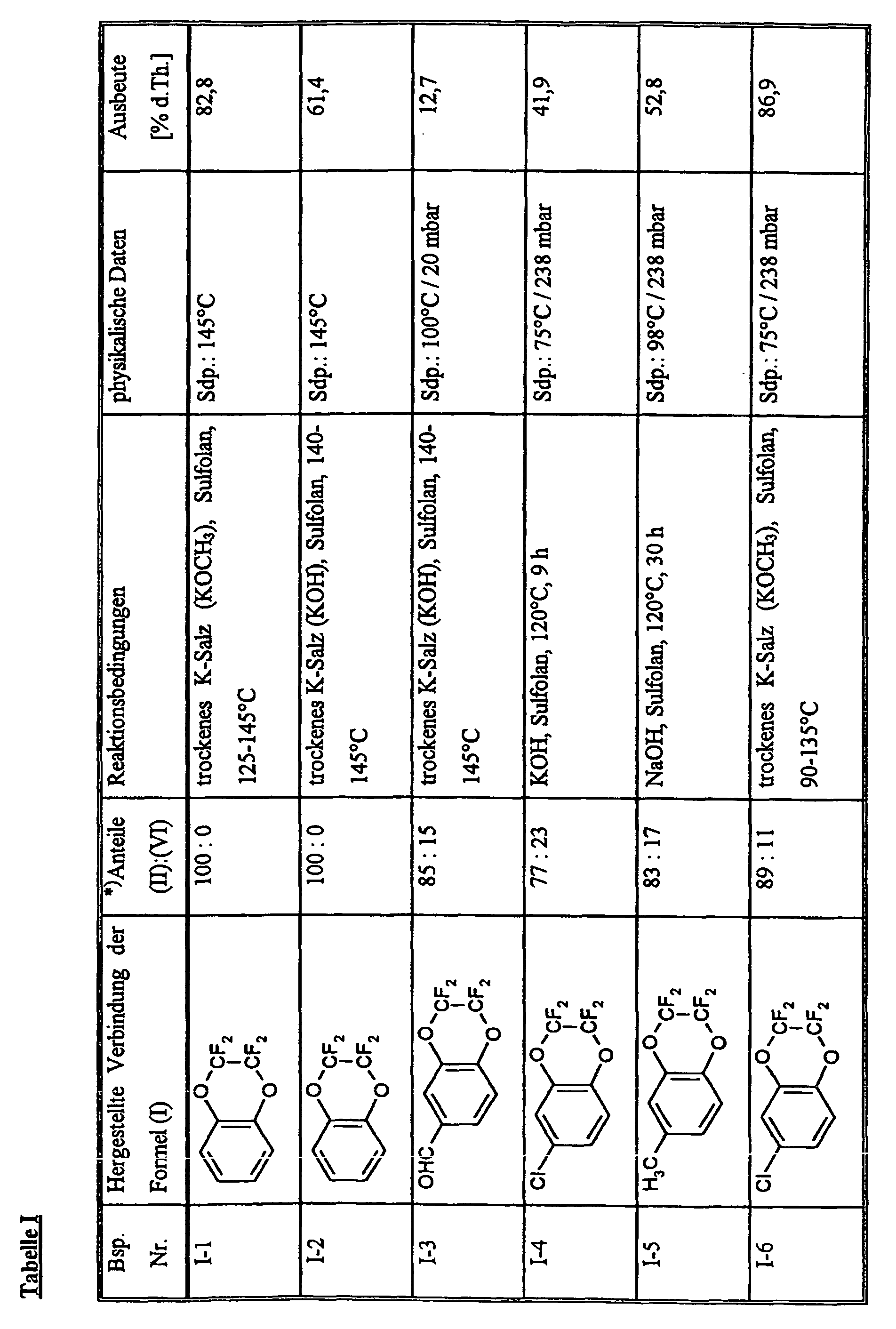

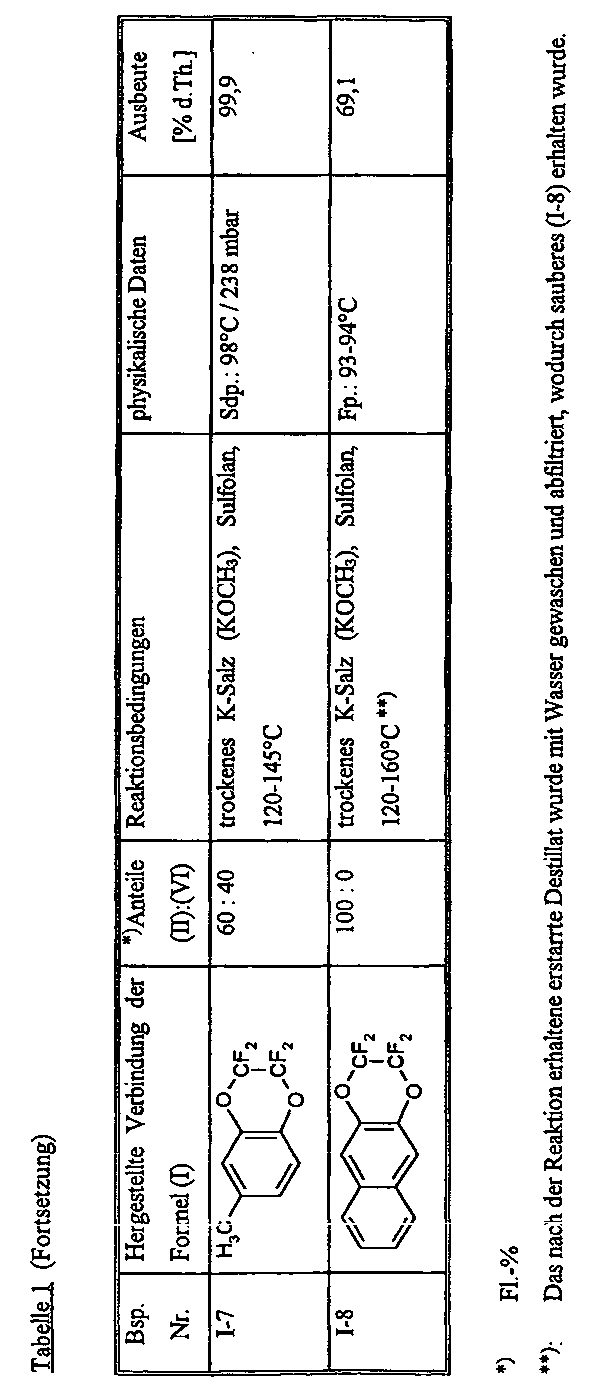

### Beispiel II-1

### Herstellung von 2-(2-Brom-1,1,2,2-tetrafluorethoxy)-phenol

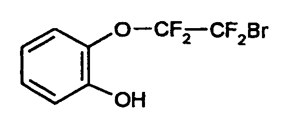

Eine Lösung von 54,42 g 1-(2-Brom-1,1,2,2-tetrafluorethoxy)-2-methoxybenzol in 500 ml eines Säuregemisches Essigsäure (96 gew.-%ig) / Bromwasserstoffsäure (48 gew-%ig) 2,5 : 1 wurde 43 Stunden unter Rückfluß gerührt, wobei die Vollständigkeit der Reaktion per Gaschromatographie (GC) verfolgt wurde. Danach wurde die Reaktionsmischung auf 20°C abgekühlt und auf Wasser gegossen. Die organische Phase wurde abgetrennt und die wäßrige Phase zweimal mit Dichlormethan extrahiert. Die Extrakte wurden zur organischen Phase gegeben, über Magnesiumsulfat getrocknet und im Vakuum eingedampft. Die Destillation des Rückstands ergab 43,55 g (83,9 % d. Th.) 2-(2-Brom-1,1,2,2-tetrafluorethoxy)-phenol mit einem Siedepunkt von 84°C/20 mbar.

| C/H-Analyse [%] | C | H |
|---|---|---|
| ber. für C₈H₅O₂BrF₄ | 33,24 | 1,74 |
| gef. | 33,4 | 1,7 |

### Beispiel II-2

### Herstellung von 2-(2-Brom-1,1,2,2-tetrafluorethoxy)-3-hydroxynaphthalin und 2-(1,1,2,2-tetrafluorethoxy)-3-hydroxynaphthalin

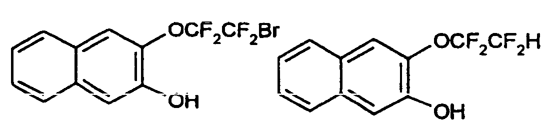

2,68 g einer Mischung aus 50 Fl.-% 2-(2-Brom-1,1,2,2-tetrafluorethoxy)-3-methoxy-naphthalin und 50 Fl.-% 2-(1,1,2,2-Tetrafluorethoxy)3-methoxynaphthalin wurden für 30 Stunden in 65 ml einer Lösung aus Essigsäure (96 gew.-%ig) und. Bromwasserstoffsäure (48 gew.-%ig) im Volumenverhältnis 2,5 : 1 bei 120°C gerührt, wobei die Vollständigkeit der Reaktion per Gaschromatographie (GC) verfolgt wurde. Danach wurde die Reaktionsmischung auf 20°C abgekühlt, auf Wasser gegossen und mit Dichlormethan extrahiert. Die vereinigten organischen Phasen wurden über Magnesiumsulfat getrocknet. Das Lösungsmittel wurde im Vakuum abgedampft und der Rückstand von 1,60 g, der aus 63 Fl.%- 2-(2-Brom-1,1,2,2-tetrafluorethoxy)-3-hydroxynaphthalin und 37 Fl.-% 2-(1,1,2,2-Tetrafluorethoxy)-3-hydroxynaphthalin bestand (GC-Analyse), durch Säulen chromatographie an 97 g Kieselgel mit Hexan/Dichlormethan 1 : 1,9 als Laufmittel aufgetrennt. Es wurden 0,71 g (55,5 % d.Th.) der bromhaltigen Verbindung vom Schmelzpunkt 67-68°C und 0,532 g (42 % d.Th.) der bromfreien Verbindung vom Schmelzpunkt 70-71°C erhalten. Proben für die Analyse wurden durch Sublimation bei 60°C/10 mbar erhalten.

| bromhaltige Verbindung: C/H-Analyse [%] | C | H |
|---|---|---|
| ber. für C₁₂H₇O₂BrF₄ | 42,50 | 2,08 |
| gef. | 42,4 | 2,1 |

| bromhaltige Verbindung: C/H-Analyse [%] | C | H |
|---|---|---|
| ber. für C₁₂H₈O₂F₄ | 55,39 | 3,10 |
| gef | 55,2 | 3,2 |

Die oben beschriebenen Beispiele und weitere, die analog zu diesen durchgeführt wurden, sind in Tabelle II zusammengefaßt.

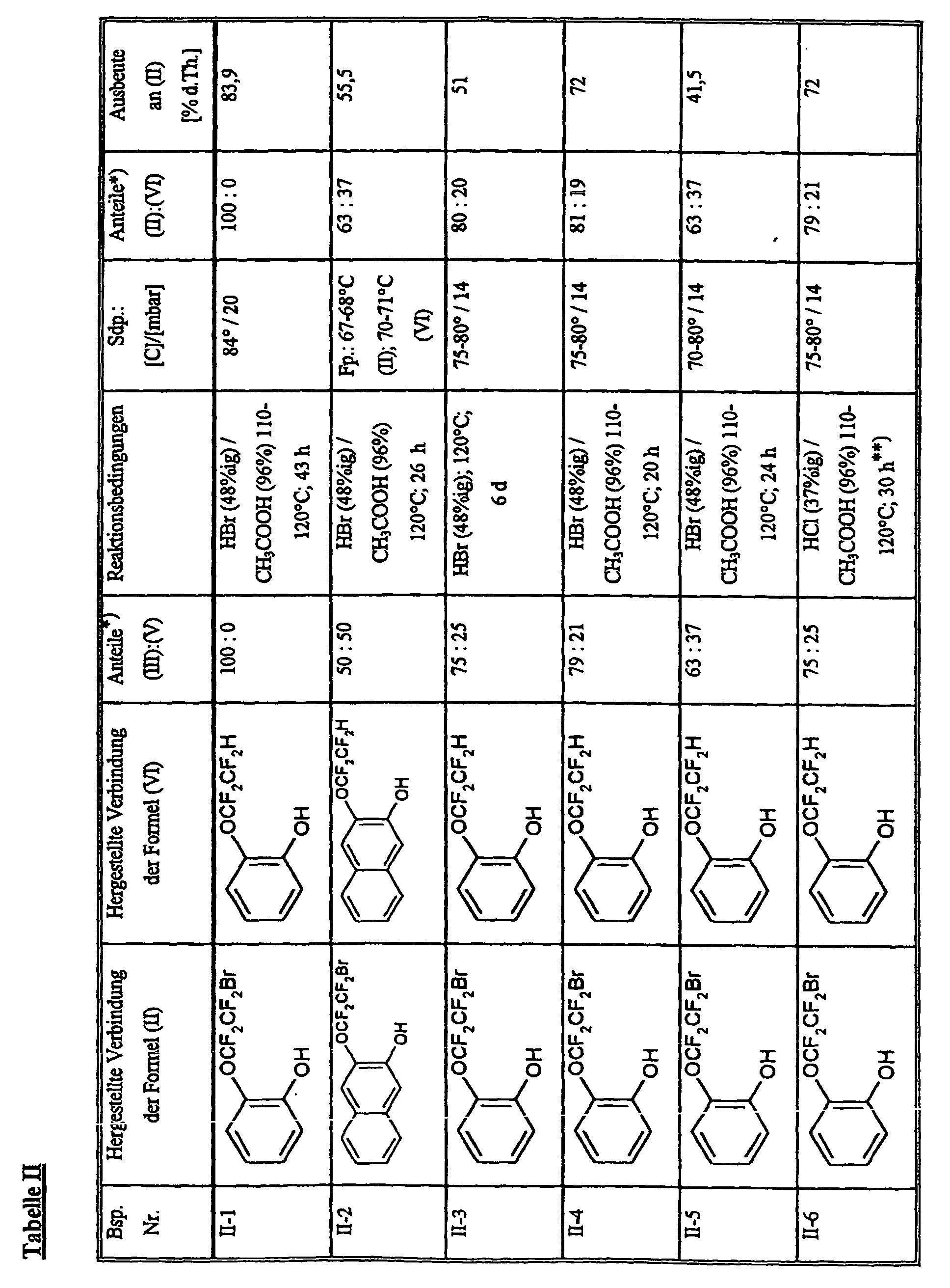

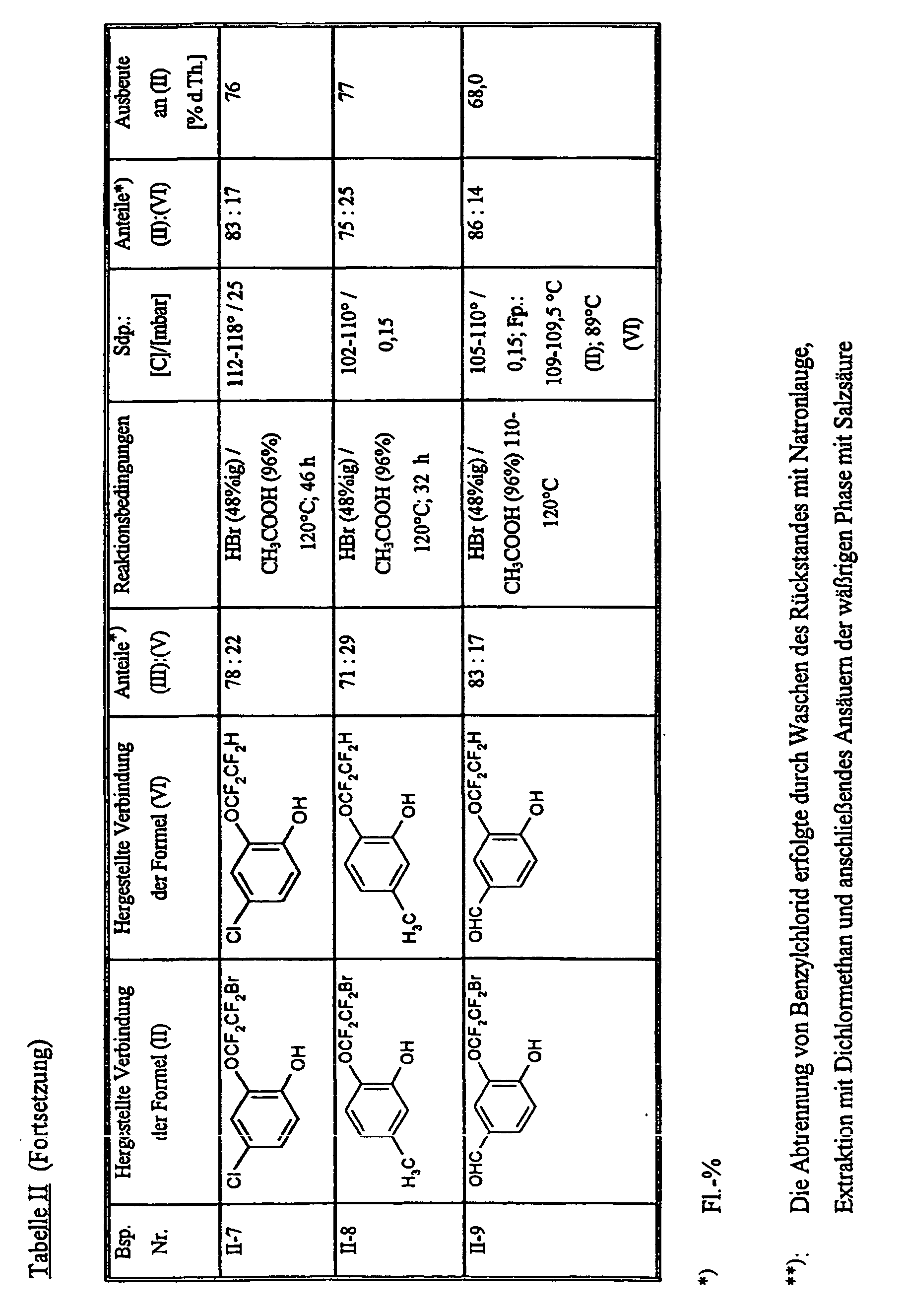

### Beispiel III-1

### Herstellung von 2-(2-Brom-1,1,2,2-tetrafluorethoxy)-anisol

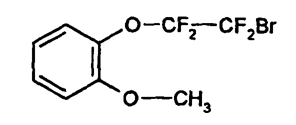

124,8 g 1,2-Dibromtetrafluorethan wurden unter Stickstoff tropfenweise zu einer gerührten Mischung von 30 g Guajacol, 50 g wasserfreiem Kaliumcarbonat und 450 ml trockenem Dimethylsulfoxid (DMSO) gegeben. Danach wurde die Mischung auf 100°C erwärmt und 24 Stunden bei dieser Temperatur gerührt. Dann wurde die Reaktionsmischung auf 20°C gekühlt und auf Wasser gegossen. Die organische Phase wurde abgetrennt und die wäßrige Phase zweimal mit Dichlorethan extrahiert. Die Extrakte wurden zur organischen Phase gegeben und über Magnesiumsulfat getrocknet. Das Lösungsmittel wurde dann im Vakuum abgedampft und der Rückstand destilliert. Es wurden, bei 80 - 98°C/14 mbar übergehend, 51,0 g eines Gemisches aus 83 Fl.-% 1-(2-Brom- 1,1,2,2-tetrafluorethoxy)-2-methoxybenzol (58,2 % d.Th) und 17 Fl.-% 1-(1,1,2,2-Tetrafluorethoxy)-2-methoxybenzol erhalten (GC-, ¹H und ¹⁹F-NMR-Analyse). Das gewünschte Produkt wurde durch fraktionierte Destillation über eine längere Füllkörperkolonne rein erhalten.
Sdp.: 80 - 82°C / 10 mbar

| C/H-Analyse [%] | C | H |
|---|---|---|
| ber. für C₉H₇O₂BrF₄ | 35,67 | 2,33 |
| gef. | 35,8 | 2,3 |

### Beispiel III-2

### Herstellung von 2-(2-Brom-1,1,2,2-tetrafluorethoxy)-anisol

30,0 g Gujacol, 70,0 g 1,2-Dibromtetrafluorethan, 50,0 g wasserfreies Kaliumcarbonat und 450 ml trockenes DMSO wurden in einen Rührautoklaven aus Edelstahl gegeben. Nach dem Spülen mit Argon wurde der Autoklav verschlossen und anschließend auf 100°C aufgeheizt. Nach 24 Stunden Rühren bei dieser Temperatur wurde auf 20°C abgekühlt, die Reaktionsmischung auf Wasser gegeben und mit Dichlormethan extrahiert. Die vereinigten Extrakte wurden über Magnesiumsulfat getrocknet. Das Dichlormethan wurde dann im Vakuum abgedampft und der Rückstand destilliert. Bei 80 - 95°C/14 mbar übergehend, wurden 62,3 g eines Gemisches aus 70 Fl.-% 1-(2-Brom-1,1,2,2-tetrafluor-ethoxy)-2-methoxybenzol (59,2 % d.Th) und 30 Fl.-% 1-(1,1,2,2-Tetrafluorethoxy)-2-methoxybenzol erhalten (GC-Analyse).

### Beispiel III-3

### Herstellung von 2-(2-Brom-1,1,2,2-tetrafluorethoxy)-3-methoxynaphthalin und 2-(1,1,2,2-Tetrafluorethoxy)-3-methoxynaphthalin

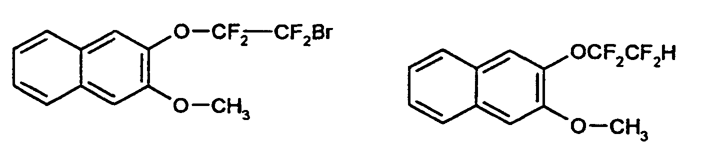

6,0 g 3-Methoxy-2-naphthol, 21,75 g 1,2-Dibromtetrafluorethan und 5,0 g wasserfreiem Kaliumcarbonat wurden in 400 ml trockenem DMSO analog zu Beispiel III-1 66 Stunden bei 100°C umgesetzt. Nach der Reaktion wurde das Lösungsmittel im Vakuum abgedampft (67°C/10 mbar). Der Rückstand wurde mit Wasser vermengt und die Mischung mit Dichlormethan extrahiert. Die organische Phase wurde abgetrennt, über Magnesiumsulfat getrocknet und zur Trockene eingedampft. Das Rohprodukt (10,0 g), bestehend aus je 50 Gew.-% 2-(2-Brom-1,1,2,2-tetrafluorethoxy)-3-methoxynaphthalin und 2-(1,1,2,2-Tetrafluorethoxy)-3-methoxynaphthalin (GC, ¹H- u. ¹⁹F-NMR-Analyse), wurde durch Säulenchromatographie an 360 g Kieselgel (Eluent: Hexan/Dichlormethan 10 : 3) getrennt. Man erhielt 2,6 g (21,4 % d.Th.) des gewünschten bromhaltigen Produkts 1,7 g (18 Fl.-% d.Th.) der entsprechenden bromfreien Verbindung und 2,7g einer 1:1-Mischung bei der (GC-Analyse). Damit betrugen die Gesamtausbeuten von an bromhaltiger und bromfreier Verbindung ungefähr je 33 % d.Th..

| | |
|---|---|
| Sdp. (bromhaltige Verbindung) | 92°C / 0,08 mbar |
| Sdp. (bromfreie Verbindung) | 82°C / 0,1 mbar |

Die oben beschriebenen Beispiele und weitere, die analog zu diesen durchgeführt wurden, sind in Tabelle III zusammengefaßt.

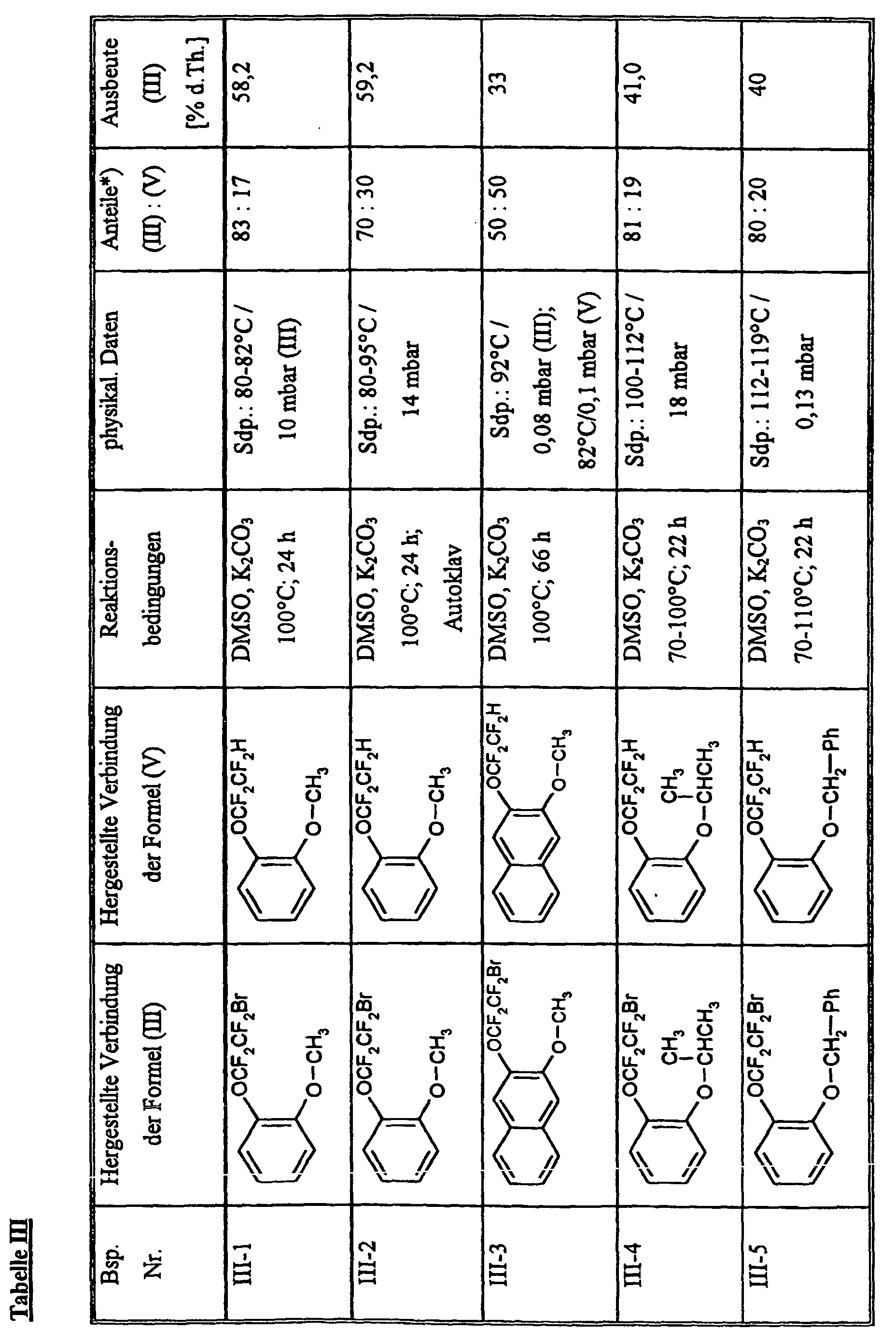

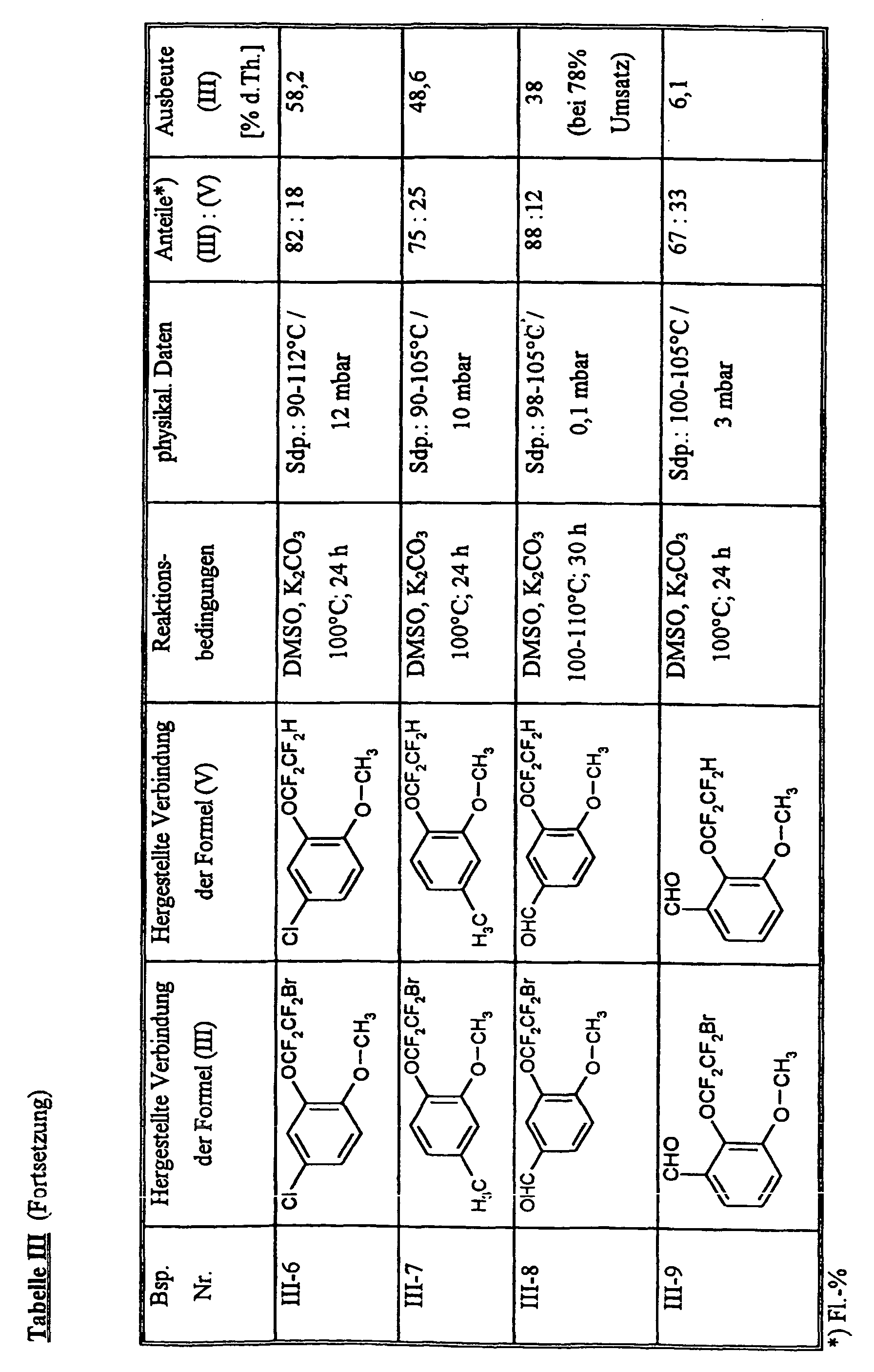

## Patentansprüche

1. Verfahren zur Herstellung von 2,2,3,3-Tetrafluor-1,4-benzodioxanen der Formel 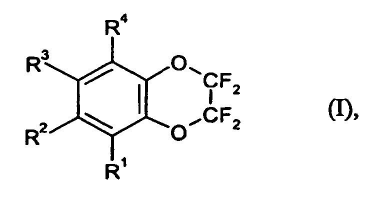 in welcher
R¹ bis R⁴ unabhängig voneinander jeweils für Wasserstoff, Halogen oder C₁- C₆-Alkyl stehen,
wobei zwei benachbarte Reste von R¹ bis R⁴ auch gemeinsam für - (CH₂)₃-, -(CH₂)₄-, -(CH₂)₅- oder eine gegebenenfalls durch Halogen oder C₁-C₆-Alkyl substituierte Kette -CH=CH-CH=CH- stehen können,
und wobei ein oder zwei der Reste R¹ bis R⁴ darüber hinaus auch für Nitro, Cyano, -CO-R⁶ oder -C(OR⁷)₂R⁶ stehen können, mit R⁶ = Wasserstoff oder C₁-C₆-Alkyl und R⁷ = C₁-C₆-Alkyl oder beide Reste gemeinsam = -CH₂CH₂-, -CH₂CH(CH₃)-, CH₂CH(C₂H₅)- oder -CH(CH₃)CH(CH₃)-,
**dadurch gekennzeichnet, daß** man o-(2-Brom-tetrafluorethoxy)-phenole der Formel 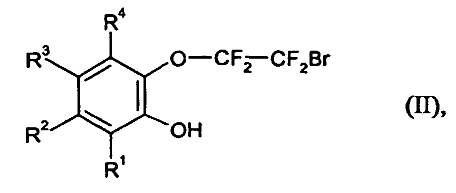
in welcher R¹ bis R⁴ die bei Formel (I) angegebene Bedeutung haben,
in Gegenwart eines Säurebindemittels cyclokondensiert.

2. Verfahren zur Herstellung der Phenole der Formel (II) des Anspruchs 1, **dadurch gekennzeichnet, daß** man Verbindungen der Formel (III) 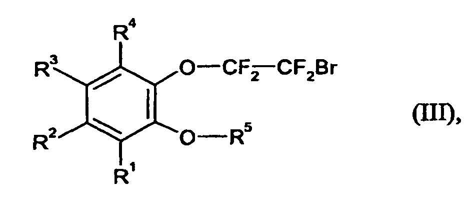 in welcher
R¹ bis R⁴ die bei Formel (I) angegebene Bedeutung haben und
R⁵ für C₁-C₆-Alkyl oder Benzyl steht,
einer Etherspaltung unterwirft.

3. Verfahren zur Herstellung der Verbindungen der Formel (III) des Anspruchs 2, **dadurch gekennzeichnet, daß** man Verbindungen der Formel (IV) 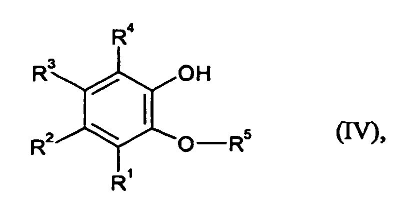
in welcher R¹ bis R⁴ die bei Formel (I) in Anspruch 1 und R⁵ die bei Formel (III) in Anspruch 2 angegebenen Bedeutungen haben,
mit 1,2-Dibrom-1,1,2,2-tetrafluorethan in Gegenwart eines Säurebindemittels umsetzt.

4. Verfahren zur Herstellung von 2,2,3,3-Tetrafluor-1,4-benzodioxanen der Formel 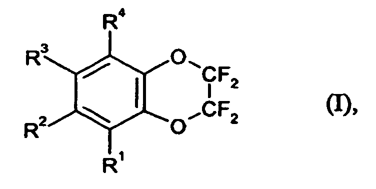 in welcher
R¹ bis R⁴ unabhängig voneinander jeweils für Wasserstoff, Halogen oder C₁- C₆-Alkyl stehen,
wobei zwei benachbarte Reste von R¹ bis R⁴ auch gemeinsam für - (CH₂)₃-, -(CH₂)₄-, -(CH₂)₅- oder eine gegebenenfalls durch Halogen oder C₁-C₆-Alkyl substituierte Kette -CH=CH-CH=CH- stehen können
und wobei ein oder zwei der Reste R¹ bis R⁴ darüber hinaus auch für Nitro, Cyano, -CO-R⁶ oder C(OR⁷)₂R⁶ stehen können, mit R⁶ = Wasserstoff oder C₁-C₆-Alkyl und R⁷ = C₁-C₆-Alkyl oder beide Reste gemeinsam = -CH₂CH₂-, -CH₂CH(CH₃)-, -CH₂CH(C₂H₅)- oder -CH(CH₃)CH(CH₃)-,
**dadurch gekennzeichnet, daß** man Verbindungen der Formel (IV) 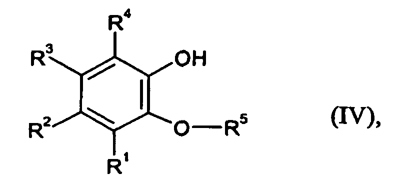
in welcher R¹ bis R⁴ die bei Formel (I) in Anspruch 1 und R⁵ die bei Formel (III) in Anspruch 2 angegebenen Bedeutungen haben,
mit 1,2-Dibrom-1,1,2,2-tetrafluorethan in Gegenwart eines Säurebindemittels umsetzt, so eine Verbindung der Formel (III) erhält 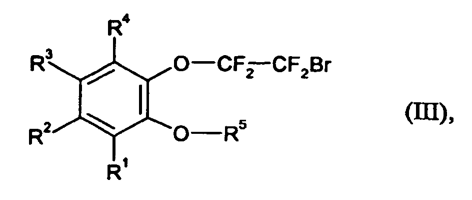 in welcher
R¹ bis R⁴ die bei Formel (I) in Anspruch 1 angegebene Bedeutung haben und
R⁵ für C₁-C₆-Alkyl oder Benzyl steht,
diese einer Etherspaltung unterwirft, so eine Verbindung der Formel (II) erhält 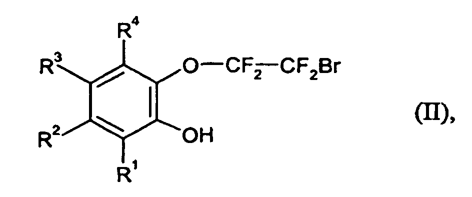 in welcher R¹ bis R⁴ die bei Formel (I) in Anspruch 1 angegebene Bedeutung haben,
und diese in Gegenwart eines Säurebindemittels cyclokondensiert.

5. Verbindungen der Formel 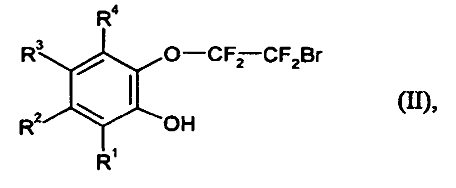
in welcher R¹ bis R⁴ die bei Formel (I) in Anspruch 1 angegebene Bedeutung haben.

6. Verbindungen der Formel 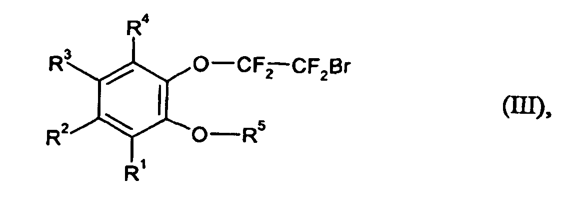 mit Ausnahme von **1-(2-Brom-1,1,2,2-tetrafluorethoxy)-2-alkoxy-4-nitrobenzolen**

## Claims

1. Process for preparing 2,2,3,3-tetrafluoro-1,4-benzodioxanes of the formula 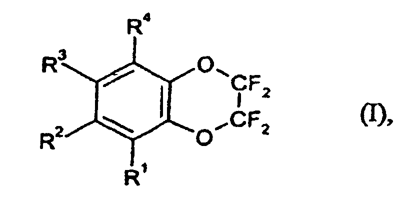 where
R¹ to R⁴ are each independently hydrogen, halogen or C₁-C₆ alkyl,
or where two adjacent radicals of R¹ to R⁴ together form a group of the formula -(CH₂)3-, -(CH₂)₄-, -(CH₂)₅- or a group of the formula -CH=CH-CH=CH- which is unsubstituted or substituted by halogen or C₁-C₆-alkyl,
and where one or two of the radicals R¹ to R⁴ may additionally also be nitro, cyano, -CO-R⁶ or -C(OR⁷)₂R⁶, where R⁶ is hydrogen or C₁-C₆-alkyl and R⁷ = C₁-C₆-alkyl, or both radicals together = -CH₂CH₂-, -CH₂CH(CH₃)-, -CH₂CH(C₂H₅)- or -CH(CH₃)CH(CH₃)-,
**characterized in that** o-(2-bromotetrafluoroethoxy) phenols of the formula 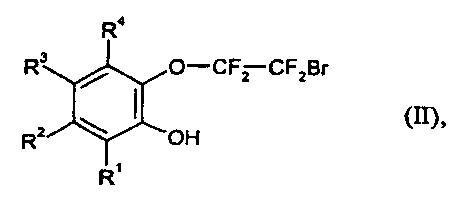 wherein R¹ to R⁴ are as defined for formula (I)
are cyclocondensed in the presence of an acid binder.

2. Process for preparing the phenols of the formula (II) of Claim 1, **characterized in that** compounds of the formula (III) 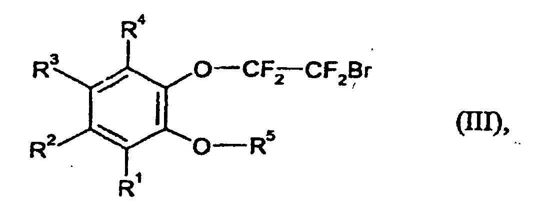 wherein
R¹ to R⁴ are each as defined for formula (I) and
R⁵ is C₁-C₆-alkyl or benzyl
are subjected to an ether cleavage.

3. Process for preparing the compounds of the formula (III) of Claim 2, **characterized in that** compounds of the formula (IV) 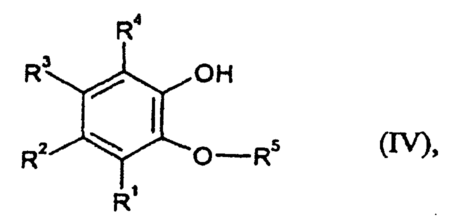 wherein R¹ to R⁴ are as defined in Claim 1 for formula (I) and R⁵ is as defined in Claim 2 for formula (III)
are reacted with 1,2-dibromo-1,1,2,2-tetrafluoroethane in the presence of an acid binder.

4. Process for preparing 2,2,3,3-tetrafluoro-1,4-benzodioxanes of the formula 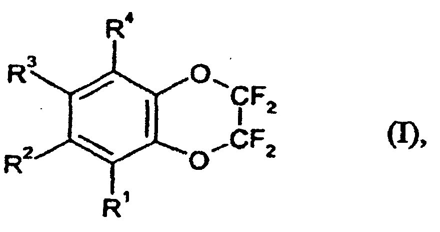 wherein
R¹ to R⁴ are each independently hydrogen, halogen or C₁-C₆ alkyl, or where two adjacent radicals of R¹ to R⁴ together form a group of the formula -(CH₂)₃-, -(CH₂)₄-, -(CH₂)₅- or a group of the formula -CH=CH-CH=CH- which is unsubstituted or substituted by halogen or C₁-C₆-alkyl,
and where one or two of the radicals R¹ to R⁴ may additionally also be nitro, cyano, -CO-R⁶ or -C(OR⁷)₂R⁶, where R⁶ is hydrogen or C₁-C₆-alkyl and R⁷ = C₁-C₆-alkyl, or both radicals together = -CH₂CH₂-, -CH₂CH(CH₃)-, -CH₂CH(C₂H₅)- or -CH(CH₃)CH(CH₃)-,
**characterized in that** compounds of the formula (IV) 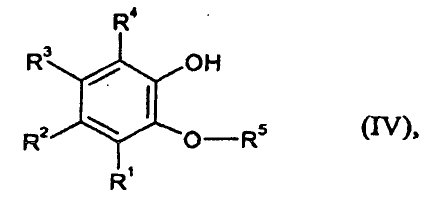 wherein R¹ to R⁴ are as defined in Claim 1 for formula (I) and R⁵ is as defined in Claim 2 for formula (III)
are reacted with 1,2-dibromo-1,1,2,2-tetrafluoroethane in the presence of an acid binder to obtain a compound of the formula (III) 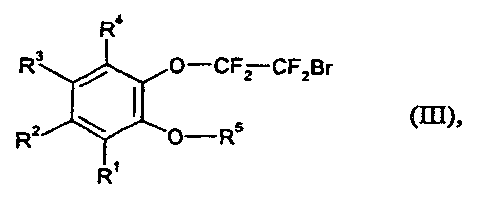 wherein
R¹ to R⁴ are each as defined in Claim 1 for formula (I) and
R⁵ is C₁-C₆-alkyl or benzyl,
the latter is subjected to an ether cleavage to obtain a compound of the formula (II) 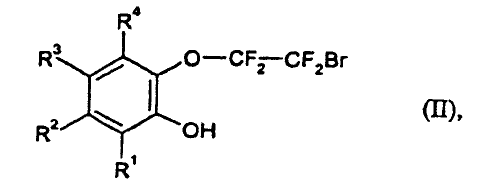 in which R¹ to R⁴ are as defined in Claim 1 for formula (I),
and the latter is cyclocondensed in the presence of an acid binder.

5. Compounds of the formula 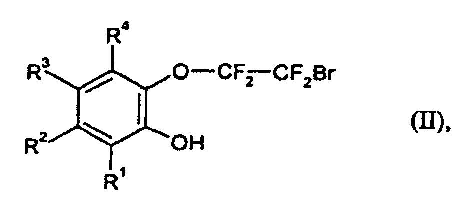 wherein R¹ to R⁴ are as defined in Claim 1 for formula (I).

6. Compounds of the formula 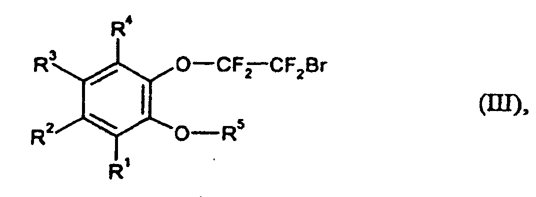 excluding **1-(2-bromo-1,1,2,2-tetrafluoroethoxy)-2-alkoxy-4-nitrobenzenes.**

## Revendications

1. Procédé pour la préparation des 2,2,3,3-tétrafluoro-1,4-benzodioxannes de formule 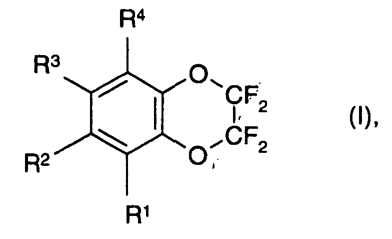 dans laquelle
R¹ à R⁴ représentent chacun, indépendamment les uns des autres, l'hydrogène, un halogène ou un groupe alkyle en C₁-C₆,
deux symboles voisins, parmi R¹ à R⁴, pouvant également représenter ensemble un groupe -(CH₂)₃-, -(CH₂)₄-, -(CH₂)₅- ou une chaîne -CH=CH-CH=CH- éventuellement substituée par des halogènes ou des groupes alkyles en C₁-C₆,
un ou deux des symboles R¹ à R⁴ pouvant également représenter un groupe nitro, cyano, -CO-R⁶ ou -C(OR⁷)₂R⁶, R⁶ représentant l'hydrogène ou un groupe alkyle en C₁-C₆ et R⁷ un groupe alkyle en C₁-C₆ ou bien R⁶ et R⁷, ensemble : -CH₂CH₂-, -CH₂CH(CH₃)-, -CH₂CH(C₂H₅)- ou -CH(CH₃)CH(CH₃)-,
**caractérisé en ce que** l'on soumet des o-(2-bromo-tétrafluoréthoxy)-phénols de formule 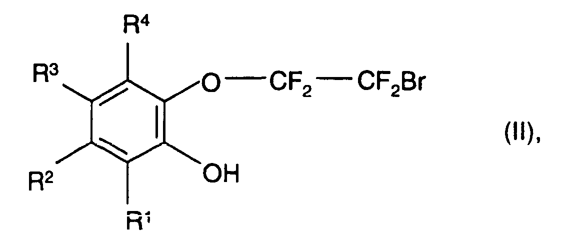 dans laquelle R¹ à R⁴ ont les significations indiquées en référence à la formule (I),
à cyclocondensation en présence d'un capteur d'acide.

2. Procédé pour la préparation des phénols de formule (II) de la revendication 1, **caractérisé en ce que** l'on soumet des composés de formule (III) 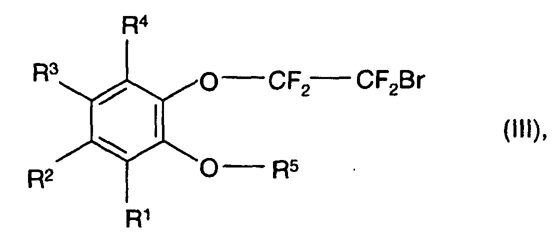 dans laquelle
R¹ à R⁴ ont les significations indiquées en référence à la formule (I) et
R⁵ représente un groupe alkyle en C₁-C₆ ou benzyle, à scission de la fonction éther.

3. Procédé pour la préparation des composés de formule (III) de la revendication 2, **caractérisé en ce que** l'on fait réagir des composés de formule (IV) 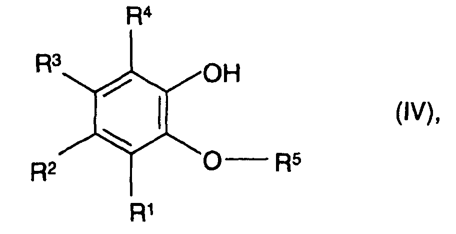 dans laquelle R¹ à R⁴ ont les significations indiquées en référence à la formule (I) dans la revendication 1 et R⁵ les significations indiquées en référence à la formule (III) dans la revendication 2,
avec le 1,2-dibromo-1,1,2,2-tétrafluoréthane en présence d'un capteur d'acide.

4. Procédé pour la préparation des 2,2,3,3-tétrafluoro-1,4-benzodioxannes de formule 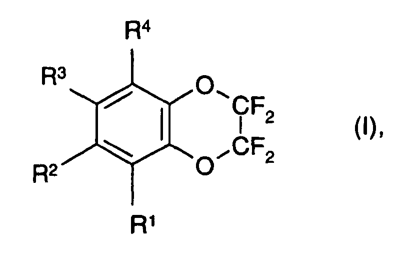 dans laquelle
R¹ à R⁴ représentent chacun, indépendamment l'un de l'autre, l'hydrogène, un halogène ou un groupe alkyle en C₁-C₆,
deux symboles voisins, parmi R¹ à R⁴, pouvant également représenter ensemble un groupe -(CH₂)₃-, -(CH₂)₄-, -(CH₂)₅- ou une chaîne -CH=CH-CH=CH- éventuellement substituée par des halogènes ou des groupes alkyles en C₁-C₆,
un ou deux des symboles R¹ à R⁴ pouvant également représenter un groupe nitro, cyano, -CO-R⁶ ou -C(OR⁷)₂R⁶, R⁶ représentant l'hydrogène ou un groupe alkyle en C₁-C₆ et R⁷ un groupe alkyle en C₁-C₆ ou bien, les deux ensembles, représentant -CH₂CH₂-, -CH₂CH(CH₃)-, -CH₂CH(C₂H₅)- ou -CH(CH₃)CH(CH₃)-,
**caractérisé en ce que** l'on fait réagir des composés de formule (IV) 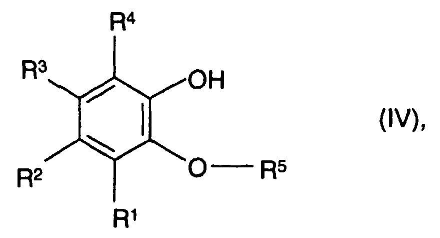 dans laquelle R¹ à R⁴ ont les significations indiquées en référence à la formule (I) dans la revendication 1 et R⁵ les significations indiquées en référence à la formule (III) dans la revendication 2,
avec le 1,2-dibromo-1,1,2,2-tétrafluoréthane en présence d'un capteur d'acide, la réaction donnant un composé de formule (III) 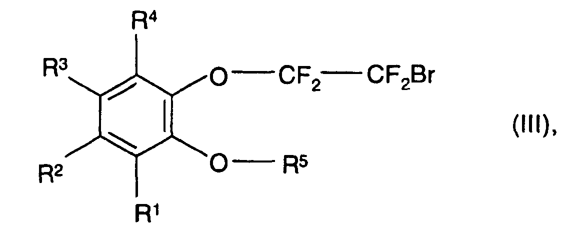 dans laquelle
R¹ à R⁴ ont les significations indiquées en référence à la formule (I) dans la revendication 1 et
R⁵ représente un groupe alkyle en C₁-C₆ ou benzyle,
qu'on soumet à scission de la fonction éther, ce qui donne un composé de formule (II) 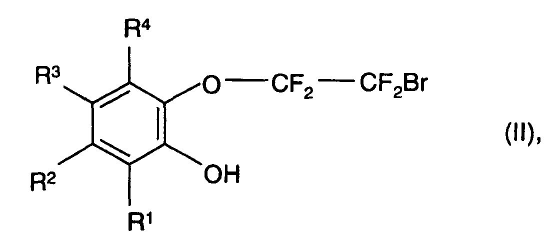 dans laquelle R¹ à R⁴ ont les significations indiquées en référence à la formule (I) dans la revendication 1, qu'on soumet à cyclocondenssation en présence d'un capteur d'acide.

5. Composés de formule 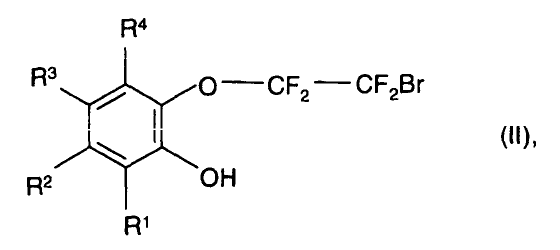 dans laquelle R¹ à R⁴ ont les significations indiquées en référence à la formule (I) dans la revendication 1.

6. Composés de formule 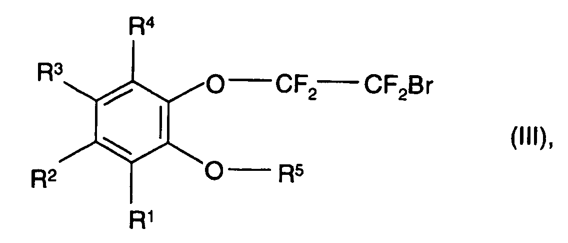 à l'exception des 1-(2-bromo-1,1,2,2-tétrafluoréthoxy)-2-alcoxy-4-nitrobenzènes.
